# EUROPEAN PATENT APPLICATION

(11) **EP 3 932 412 A1**
(43) Date of publication of application: **05.01.2022**
(21) Application number: 20183324.1
(22) Date of filing: 30.06.2020
(51) Int. Cl.: A61K 31/737, A61P 31/14, A61K 36/03, A61K 9/08

(54) **ANTIVIRAL PHARMACEUTICAL COMPOSITION**

(71) Applicant: GSK Consumer Healthcare SARL, 1260 Nyon (CH)
(72) Inventor: Lombardi, Maria Stella, 1260 Nyon (CH); Bulsara, Pallav, Warren, New Jersey 07059 (US)
(74) Representative: Gundel, Isabelle

(57) **Abstract**

Pharmaceutical compositions are described comprising fucoidan for use in a method for the treatment or the prevention, by intranasal administration, of coronavirus infection in a human. Particularly, the viral infection is OC43 or SARS-CoV-1 or SARS-CoV-2 infection. There are also described Pharmaceutical compositions comprising fucoidan for use in a method for prevention coronavirus spread in a human population, wherein subjects of the population are tested for infection with OC43 or SARS-CoV-1 or SARS-CoV-2 and infected subjects are treated with intranasal administration of the Pharmaceutical composition.

## Description

### FIELD OF THE INVENTION

The present invention relates to pharmaceutical compositions for use in methods of treatment and prevention of infection of the respiratory tract by coronaviruses, particularly the coronavirus species OC43, SARS-CoV-1 and SARS-CoV-2. The present invention further relates to the control of the spread of infection with coronaviruses, particularly the coronavirus species OC43 and SARS-CoV-2.

### BACKGROUND TO THE INVENTION

Acute respiratory viral infection (ARVI) is an acute infection of the respiratory tract, present in the form of catarrhal inflammation of the upper respiratory airway, and progressing with fever, runny nose, sneezing, cough and sore throat. The illness disturbs the general health to different extents. It may end in spontaneous recovery but may also cause severe illness and complications even leading to death of the patient. ARVIs can be provoked by more than 200 species of respiratory viruses. Human coronavirus species are responsible for about 15% of ARVIs in humans. Coronaviruses consist of an enveloped single strand positive sense RNA genome of 26 to 32 kb in length. They are classified by phylogenetic similarity into four categories: a (e.g. 229E and NL-63), β (e.g. SARS-CoV-2, SARS-CoV, MERS-CoV and OC43), γ and δ. SARS-CoV-2 has been reported to have 79% sequence identity to SARS-CoV, however certain regions of the SARS-CoV-2 genome exhibit greater or lesser degrees of conservation to SARS-CoV. Human coronaviruses comprise species such as OC43 which generally cause less severe illness. Aggressive species of coronavirus comprise SARS-CoV, MERS-CoV and SARS-CoV-2, which can cause severe symptoms and critical conditions and complications with high mortality rate such as the severe acute respiratory syndrome (SARS) and acute respiratory distress syndrome (ARDS).

Modes of infection and reproduction differ greatly between the different families of respiratory viruses. Current antiviral drugs for ARVI treatment target key molecules in binding, endocytosis, fusion (uncoating), DNA or RNA synthesis and replication, assembly and release of the viruses. Consequently, they are effective against a specific family of viruses that uses those target molecules. Even within one virus family and species, effectiveness may vary due to different mechanisms, genotypes and expression of the target proteins. Due to innate or acquired mutation, different strains of viruses may be less susceptible or even resistant to drug treatment. However, patients do often not have the possibility to get tested for viral infection, so that it is not always possible to select an appropriate antiviral treatment. This is because most tests for ARVI today require taking of a nasal or pharyngeal swab in a clinical setting and analysis of the obtained specimen in a laboratory. Testing in clinical setting, laboratory capacity and testing equipment are limited. Especially in a pandemic situation the demand for testing cannot be satisfied. It is known that asymptomatic and presymptomatic carriers of the virus can contribute to the spread of the virus, and there is thus a need for a treatment that can be used as a preventive treatment or at least when first symptoms occur, irrespective of test results. Preventive treatment with antiviral drugs is however not favoured broadly, namely due to potential side-effects, imminent development of drug resistance and high cost. Currently available antiviral drugs are generally administered systemically, such as per oral or intravenous administration. This results in systemic concentrations of the drug that may lead to side effects. Therefore, local treatment for ARVI that effectively stops and kills the virus at the initial site of infection, the respiratory mucosa, is highly desirable. There is also a need for pharmaceutical compositions that can be used to prevent ARVI in humans and are easy to use, and with a low risk of side-effects.

In December 2019, a new coronavirus species was observed as a human ARVI pathogen for the first time. The pathogen was later named severe acute respiratory syndrome coronavirus 2 (SARS-CoV-2) and the disease caused by the virus in humans was named coronavirus disease (COVID-19). The virus, via human-to-human infection, has spread to all parts of the world leading to the classification as a pandemic in March 2020 and presents a major global health threat. Currently, clinical studies test the effectiveness of re-designated approved or pipeline antiviral drugs for treatment of infection with SARS-CoV-2 or COVID-19. However, the effectiveness of those candidates is not established yet, and no preventive treatment or vaccine is currently available. One re-purposed pipeline drug is the antiviral drug remdesivir, a prodrug of an adenosine nucleoside triphosphate analogue. It was suggested as an investigational drug for COVID-19 because it had generated promising results against MERS-CoV and SARS-CoV-1 in previous animal studies. Pizzorno et al. (Characterization and treatment of SARS-CoV-2 in nasal and bronchial human airway epithelia, bioRxiv, 02.04.2020, DOI: 10.1101/2020.03.31.017889) found that remdesivir reduces the relative viral production in SARS-CoV-2 infected reconstituted human airway epithelia cells. Numerous clinical studies were initiated that aim to establish if intravenous remdesivir has a clinical benefit in treatment of COVID-19. The drug was granted an Emergency Use Authorization for treatment of hospitalised adults and children with severe COVID-19 by the U.S. Food and Drug Administration in May 2020 and is the only approved drug for COVID-19 at the time of filing of the present patent application.

Liu Y et al. (Viral dynamics in mild and severe cases of COVID-19, The Lancet, Volume 395, Issue 10223, 15-21 February 2020, Pages 507-513, DOI: 10.1016/S1473-3099(20)30232-2) observed that patients with severe COVID-19 tend to have a high viral load of their nasopharyngeal swab samples and a long virus-shedding period. Authors suggest that the viral load of SARS-CoV-2 might be a useful marker for assessing disease severity and prognosis.

Hou, Y.J. et al. (SARS-CoV-2 Reverse Genetics Reveals a Variable Infection Gradient in the Respiratory Tract, Cell 11447 (2020), doi: https://doi.org/10.1016/j.cell.2020.05.042), suggest that nasal cells are the dominant initial site for SARS-CoV-2 respiratory tract infection and that the virus may be introduced to the lower respiratory tract by aspiration of nasal secretion or mucus containing a high viral load.

Fucoidan, a marine polysaccharide, has been tested against various respiratory virus families, genus, species and strains with mixed results.

WO2009/027057 teaches antiviral compositions comprising a sulphated polysaccharide and discloses that carrageenan and fucoidan inhibit the parainfluenza virus 3 mediated cell death in HNep cells when the cells were inoculated in the presence of the polysaccharides, wherein carrageenan is more effective than fucoidan (example 13). Similar results are achieved in the same experiment after inoculation with influenza virus H3N2, wherein iota-carrageenan has the best protective effect at most concentrations (example 14). The disclosure further teaches that pretreatment of the virus with fucoidan cannot reduce plaque formation of avian influenza virus H5N1 in MDCK cells (example 15).

WO2011/100805 teaches methods for inhibiting a virus of the orthomyxoviridae family comprising contacting the virus or a cell infected with the virus with an effective amount of a formulation comprising a sulfated polysaccharide having an average molecular weight of 4,000 Daltons or greater. Sulfated polysaccharide formulations reduce the cytopathic effect (CPE) of Influenza A type H1N1, strain California/07/2009 in MDCK cells, but are not effective in the same experiment against influenza A type H3N2, strain Brisbane/10/2007. The applicants of WO2011/100805 communicated on 5, March 2020 in a press release that in previous investigations they noted little activity of their commercially available fucoidan product on coronaviruses in comparison to the extent observed with other viruses such as influenza, herpes, human metapneumovirus and respiratory syncytial virus (https://www.marinova.com.au/news/fucoidan-and-novel-coronavirus/).

Wang et. al. (Wang, W., Wu, J., Zhang, X. et al. Inhibition of Influenza A Virus Infection by Fucoidan Targeting Viral Neuraminidase and Cellular EGFR Pathway. Sci Rep 7, 40760 (2017). DOI: 10.1038/srep40760) found that fucoidan from *Kjellmaniella crassifolia* reduces CPE and plaque formation in MDCK cells after infection with different H1N1 strains (PR8, Cal09 and TX09) as well as the H3N2 strain Minnesota. It was further found in a surface plasmon resonance (SPR) assay that fucoidan binds to the Neuraminidase (NA) protein of the subtypes H1N1 (Cal09) and H3N2 (Minnesota). Authors suggest that fucoidan may inhibit the entry and release process of influenza A virus through direct binding to influenza A NA.

Fucoidan is thus only effective in some virus families, and the efficacy against a certain virus cannot be predicted. Even within one family, the response varies greatly between different species and even strains. Whilst a mechanism was proposed for the effect of fucoidan in influenza A virus, fucoidan compositions have been tested in different species and strains of influenza A with mixed results. Furthermore, the proposed mechanism is limited to influenza A viruses as it is dependent on a target molecule specific to that family.

### SUMMARY OF THE INVENTION

In a first aspect, the invention is directed to a pharmaceutical composition comprising fucoidan for use in a method for the treatment or the prevention, by intranasal administration, of coronavirus infection in a human. The present inventors found that fucoidan, administered to human nasal epithelium, reduces the replication of viruses of the coronavirus family. This is unexpected and contrary to results previously communicated by the applicants of WO2011/100805. Without being bound to a specific theory, the present inventors believe that fucoidan, comprising sulfate groups negatively charged in solution and the physiological environment, interacts with positively charged surface features, such as viral surface proteins with a net positive charge. In viruses having a positive net charge in the receptor binding domain (RBD) of their receptors that bind to host cells, binding of sulfate groups of fucoidan to these positively charged moieties may hinder the virus from attaching to and entering into the human host cell. For example, fucoidan may bind to the receptor binding domain (RBD) of the S1 domain of the spike protein (S protein) of coronavirus OC-43, SARS-CoV-1 and SARS-CoV-2, which have a net positive charge.

The pharmaceutical compositions of the present invention are therefore effective in the treatment of coronavirus infection in a human. The pharmaceutical compositions achieve a reduction of the viral load, or the viral titre, of the subject. Both parameters can be determined with methods known in the art, for example quantitative real time polymerase chain reaction (RT-PCR). In one embodiment, the treatment reduces the nasal viral load of a subject. In one embodiment, the nasal viral load is determined by RT-PCR, performed on a nasal swab specimen from the subject. As a high nasal viral load of SARS-CoV-2 is a risk factor for severe forms of COVID-19, the reduction of nasal viral load can reduce the risk of severe symptoms of COVID-19. High viral loads of coronaviruses lead to a more severe inflammatory response of the patient. Exaggerated immune response is causative for the complications mentioned in the background section. Therefore, the reduction of nasal viral load may also prevent aggravation COVID-19, and development of ARDS or SARS. Furthermore, reduction of viral load in the nose may reduce the risk that a bolus of nasal secretion is aspirated into the lung and causes lower respiratory tract infection with SARS-CoV-2, in agreement with Hou, Y.J. et al (cited above).

The invention comprises a method for treating COVID-19 comprising a method of detecting viral RNA from SARS-CoV-2 from a specimen obtained from the subject and, where viral RNA is detected, a step of treating COVID-19 as described herein. The method of detecting viral RNA from SARS-CoV-2 from a specimen obtained from the subject may use a CRISPR diagnostic technique (for instance using the DECTECTR^{™} method using Cas12 (Chen et al., Science 360, 436-439 (2018), and, specifically for SARS-CoV-2detection, Broughton et al., Nat Biotechnol (2020) https://doi.org/10.1038/s41587-020-0513-4), or using the SHERLOCK^{™} method using Cas13 (Gootenberg et al., Science 356: 438-442 (2017)). Alternatively, the method of detecting viral RNA from SARS-CoV-2 from a specimen obtained from the subject may use reverse-transcriptase polymerase-chain-reaction (RT-PCR) assays or other diagnostic methods that are known in the art.

The pharmaceutical compositions of the present invention are also suitable for the prevention of coronavirus infection in humans. For prevention of coronavirus infection, the pharmaceutical compositions of the invention are administered intranasally to subjects that have not been tested for coronavirus infection, or to subjects that have been tested for coronavirus infection and the result was negative. In particular embodiments, the subject is in a high-risk category (as defined herein), a health care professional, or is a close contact of a patient infected with SARS-CoV-2 (as defined herein). The pharmaceutical compositions are effective and useful in prevention of coronavirus infection as, if applied preventively, hinder virus from infecting cells of the nasal epithelia, are not harmful to the nasal epithelia and are conveniently applied intranasally. As fucoidan is authorised as a food supplement in doses up to 500 mg per day, the compositions of the present invention and considered safe for oral intake even if swallowed and have a low risk of adverse events.

In one embodiment, the coronavirus infection is an infection with OC43, SARS-CoV-1 or SARS-CoV-2. As found by the present inventors, the compositions of the present invention are effective in reducing the viral replication of OC43 in nasal epithelia. A similar effect is expected against SARS-CoV-1 and SARS-CoV-2. The inventors believe that negatively charged sulfate groups of fucoidan interact with positively charged surface moieties, such as surface proteins, of the virus. An example for such a positively charged surface moiety is the RBD of the S1 domain of the spike protein of OC43, SARS-CoV-1 and SARS-CoV-2 which have a positive net charge of +6 for OC-43 and SARS-CoV-2 and +2 for SARS-CoV-1. The antiviral effect of the compositions of the present invention observed in studies on OC-43 may therefore be extrapolated to SARS-CoV-1 and SARS-CoV-2.

In one embodiment, the coronavirus infection is an infection with SARS-CoV-2. Methods for identifying subjects infected with SARS-CoV-2 are known in the art and are in current clinical use. In one embodiment, high-throughput sequencing or real-time reverse-transcriptase polymerase-chain-reaction (RT-PCR) assay of specimens, for example, nasal and pharyngeal swab specimens, may be used to identify subjects with active SARS-CoV-2 infection. Alternatively, CRISPR-based diagnostic techniques may be used, as mentioned above.

According to the invention, the treatment comprises intranasal administration of pharmaceutical compositions of the present invention. This comprises nasal administration of solutions, gels, creams, powders or foams. The administration can be by pipetting, dropping or spraying the of the pharmaceutical composition into the nostrils, by nebulising or atomising of the composition and inhalation of the atomised or nebulised composition or by applying of the composition to the nasal epithelia.

In one embodiment, the composition is a nasal spray, and the intranasal administration is thus the administration of a nasal spray. Administration of a nasal spray comprises inserting a nozzle into a nostril, activating a spray mechanism that expels and atomises the composition through the nozzle and into the nostril, optionally concerted inhalation and replication of the same steps with the second nostril. The resulting droplets or particles are then deposited onto the nasal epithelia. Naturally occurring ciliary beating transports the compositions to the adjacent epithelia of the nasopharynx, oropharynx and laryngopharynx. Nasal spray administration therefore is useful to bring the composition of the present invention to the initial point of infection for coronaviruses, the epithelial cells of the upper respiratory tract. This is possible without invention of an HCP and without clinical intervention. It is accomplished with a customary, easy-to-use device. Nasal sprays have a very good user compliance.

Fucoidan should in the context of the present invention be understood as marine polysaccharides comprising L-fucose monomers, partially sulfated, that can be found in and extracted from various species of brown seaweed or brown algae (i.e. the class of Phaeophyceae). Extracts from brown seaweeds including extracts comprising fucoidan are investigated for their numerous biologic activities. As discussed in the background section, fucoidan has been screened for antiviral activity *in vitro* with mixed results. Fucoidan is not harmful to the respiratory mucosa as shown in the example. Information on the chemical structure of fucoidan is provided in the definitions. The fucoidan may be derived from any species of brown seaweed such as *Undaria pinnatifida* or *Fucus vesiculosus.*

In one embodiment, the pharmaceutical composition is an aqueous solution. Aqueous solutions are preferred as the fucoidan is compatible with water as a carrier and stable in aqueous formulations. Aqueous solutions are especially preferred because they are compatible with the spray mechanism and the materials of most kinds of commercially available nasal spray devices. Aqueous solutions are also preferred because of the low risk of side effects.

In one embodiment, the pharmaceutical composition comprises 0.1% to 2.7%, more preferably 0.25 to 1.8%, most preferably 0.5 to 1% (w/w) fucoidan. The amounts as low as 0.1% are sufficient to achieve the antiviral effect in the *in vitro* model and can reasonably be expected, due to the closeness of the model to the *in vivo* situation, to be effective *in vivo.* Furthermore, the amounts can be solubilised well in aqueous carriers and form stable aqueous solutions, which is beneficial for intranasal administration. With concentrations above 2.5% (w/w) of fucoidan, solubility becomes more challenging. Furthermore, pharmaceutical compositions comprising these amounts have an appearance and odour that is acceptable for nasal administration.

In one embodiment, the pharmaceutical composition comprises a sodium chloride solution. Sodium chloride may be used as a tonicity agent. Adjusting the osmolality of pharmaceutical compositions for nasal administration is beneficial to preserve the integrity of the nasal epithelia and is well-known in the art. Sodium chloride is preferred as the ions of this salt are present ubiquitously in the human body and no adverse effects are associated with intranasal administration of sodium chloride solutions. Furthermore, sodium chloride solution is also safe for ingestion and has an acceptable taste and odour. This is important for compliance, as after nasal administration, especially nasal spray administration as discussed above, the applied dose of the composition may end up in the pharyngeal region of the subject and may get into contact with the taste buds before being swallowed. It is furthermore preferred as it is compatible with fucoidan.

In one embodiment, the aqueous solution is isotonic. Isotonic solutions are preferred as they will not, when applied to the nasal epithelia, result in osmotic pressure, negative or positive, on the epithelia. They are therefore very mild carriers for the fucoidan compositions of the present invention, that preserves the integrity of the nasal epithelia.

Preferably, the isotonic aqueous solution is a sodium chloride solution, but other agents can be used to adjust osmolality.

It should be understood that in addition to the ingredients particularly mentioned above, the formulations described herein may include other agents conventional for nasal administration, such as preservatives, pH-adjusting agents, viscosity modifiers, hydrating agents, solvents, solubilisers, decongestants such as α-sympathomimetic drugs, essential oils such as peppermint oil or cooling agents such as menthol.

In one embodiment, the pharmaceutical composition is administered intranasally twice or three times daily into each nostril of a subject. The present inventors found that administration of two doses within 24 hours, eight hours apart, is more efficient in reducing viral replication than application of a single dose within 24 hours, and it is therefore expected that an administration three times per day further increases efficacy. Consumers are used to applying nasal spray 3 times per day or every 8 hours, and even more frequent application would be less consumer-friendly.

In one embodiment, the pharmaceutical composition is administered in a dose of 3.75 mg to 10.15 mg fucoidan into each nostril of a subject per administration. This dose is believed to be effective *in vivo.*

In one embodiment, the fucoidan is fucoidan extracted from *Undaria pinnatifida.* Fucoidan extracted from *Undaria pinnatifida* was described in the literature as sulfated galactofucan because it contains high amounts of galactose, such as 40 mol%, or more, of overall neutral sugars. *Undaria pinnatifida* is a preferred source for the fucoidan for the pharmaceutical compositions of the present invention as it provides fucoidans with a high sulfate content and is abundantly available.

In one embodiment, the fucoidan has an average molecular weight of 150 kDa and a lower molecular weight cut-off of 10 kDa. This high average molecular weight provides for a complex structure of the polysaccharide that has a large surface for interaction with the surface of the virus. The large molecules are flexible in their configuration and may embrace the surface of the virus, allowing for multiple binding interactions between surface proteins of the virus and the sulfate groups of the fucoidan. The molecular weight cut-off of 10 kDa results in a higher yield of high molecular weight polymer chains. The use of high molecular weight (MW) fucoidan with the specified MW cut-off is preferred, as no uptake to systemic circulation via the mucosa is expected for high MW polymers. Even if a dose of the composition is swallowed and ingested, systemic uptake is expected to be minor, as the high molecular weight polymers are not taken up by the intestine. No systemic effects are therefore expected from intranasal administration of the pharmaceutical compositions of the invention. This is especially preferred as this reduces the risk of side effects.

In one embodiment, the fucoidan has a sulfate content of 20% to 40%. This high sulfate content comes along with a high negative charge. A high number of negatively charged residues is believed to provide particularly good interaction and bonding with the surface proteins of viruses, in particular for virus species with surface proteins with a positive net charge. An example of such a positively charged viral surface protein is the RBD of the S1 domain of the Spike protein of OC-43 which has a net charge of +6. Without being bound to a specific theory, it is believed that multiple salt bridges are formed between the positively charged viral surface proteins and the sulfate groups of fucoidan, resulting in stable binding of the surface proteins to a part of a fucoidan molecule. This binding may even result in conformational change of the surface protein, modifying its function. If the positively charged surface protein is a viral surface protein that is needed for binding to a host cell, binding of fucoidan to this surface protein may inactivate it and prevent receptor binding and entry into a host cell. The sulfate content of a specimen of fucoidan may be determined by a gravimetric method known in the art, for example acidic hydrolysis, followed by oxidation and subsequent precipitation of barium sulfate. The determined weight for sulfate is then put in relation to the weight of the specimen of fucoidan to give the sulfate content in w/w.

### BRIEF DESCRIPTION OF THE DRAWINGS

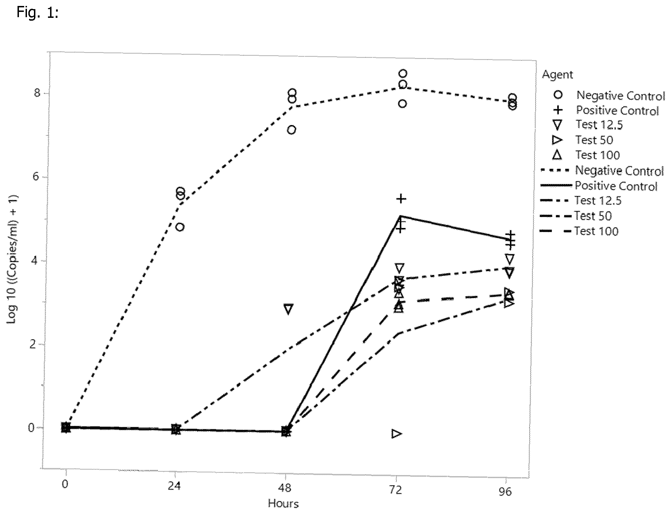

FIG. 1: OC43 viral genome copy numbers in copies/ml determined in the apical culture media over time, depending on the intervention

### DETAILED DESCRIPTION OF THE INVENTION

### DEFINITIONS

The following definitions provide the meaning that should be given to specific terms in the context of the present disclosure, unless specified otherwise in specific context.

The term fucoidan specifies marine polysaccharides comprising L-fucose monomers, partially sulfated, and extracted from edible species of brown seaweed or brown algae (Phaeophyceae). The chemical structure of fucoidans is diverse and complex. Besides L-fucose, other monosaccharides such as D-fucose, mannose, galactose, glucose and xylose may be present. The configuration of the monosaccharides can vary. Glucuronic acid may also be present. The degree of sulfation, i.e. how many of the hydroxy residues of the polysaccharide are sulfated, may vary. The sulfate substitution pattern, i.e. which hydroxy groups of a monosaccharide is sulfated, may also vary. The monosaccharides may further be partly O-acetylated. The polymer may be linear or branched. The average molecular weight (MW) of fucoidan can also vary. Average MW of 4kDa to 5MDa have been reported. The average MW of fucoidan may be determined by size exclusion chromatography (SEC) such as gel permeation chromatography (GPC).

Isotonic is a solution that has an osmolarity close to that of human plasma, i.e. from 250 to 350 mOsm/L.

Normal saline is 0.9% w/v NaCl in purified water.

The degree of sulfation is the calculated percentage of monosaccharide units that have a sulfate substitution. The degree of sulfation may be calculated from elementary analysis to obtain the percentages of carbon and sulfur atoms in a probe and putting them in relation to an amount of carbon atoms in a galactose or fucose monosaccharide (six) and the degree of acetylation determined from that probe, obtained for example with ¹H-NMR.

The sulfate content is the calculated weight percentage of sulfate groups in relation to the weight of a specimen of fucoidan. It may be determined by a gravimetric method known in the art, for example acidic hydrolysis, followed by oxidation and subsequent precipitation of barium sulfate. The determined weight for sulfate is then put in relation to the weight of the specimen of fucoidan to give the sulfate content in w/w.

High risk categories for SARS-COV-2 infection include the following: subjects of 60 years of age and over; smokers, subjects having a chronic medical condition including heart disease, lung disease, diabetes, cancer or high blood pressure; immunocompromised subjects such as subjects undergoing treatment for cancer or autoimmune diseases such as rheumatoid arthritis, systemic lupus erythematosus, multiple sclerosis and inflammatory bowel disease, subjects having a transplant and HIV positive individuals.

Close contacts of a patient infected with SARS-CoV-2 are defined as subjects living in the same household as the patient, as having had direct or physical contact the patient, or having remained within two metres of the patient for longer than 15 minutes on or after the date on which symptoms were first reported by the patient.

### EXAMPLES

### Example 1

The example describes an *in vitro* experiment that examined the effect of the pharmaceutical compositions of the invention on viral genome copy number and indicators of epithelia integrity and inflammation in a human cell model infected with human coronavirus OC43. Unless indicated otherwise, in the experiment, culture medium was MucilAir culture medium which is a ready-to-use, chemically defined, serum-free culture medium (product number EP04MM, can be purchased at Epithelix Sàrl, Geneva, Switzerland). Unless specified otherwise, incubation was at 34°C, 5% CO₂ and 100% humidity.

### Cell model

The *in vitro* model used for the experiment was a standardised nasal human 3D epithelial model called MucilAir, marketed by Epithelix Sàrl, Switzerland. The model has functional characteristics similar to *in vivo* epithelia, such as mucus production, mucociliary clearance, and secretion of cytokines and chemokines and therefore is a suitable model to support the development of new antiviral pharmaceutical compositions (B. Boda et al., Antiviral drug screening by assessing epithelial functions and innate immune responses in human 3D airway epithelium model, Antiviral Research 156 (2018) 72-79, https://doi.org/10.1016/j.antiviral.2018.06.007 and A. Pizzorno et al., Characterization and treatment of SARS-CoV-2 in nasal and bronchial human airway epithelia, bioRxiv preprint, 2020, doi: https://doi.org/10.1101/2020.03.31.017889). The mature MucilAir cell model is composed of basal cells, ciliated cells and mucus cells. The proportion of these various cell types is preserved compared to what one observes *in vivo.* The cell model used for the experiment was obtained as follows: Airway cells were obtained from patients undergoing nasal biopsy. Human airway epithelial cells were isolated, amplified and expanded by two passages to preserve the physiological characteristics of the cells. The cells were seeded onto a semi-porous membrane (Costar Transwell, pore size 0.4 µm) and cultured at the air-liquid interface for differentiation. Airway epithelia was reconstituted from a mixture of human airway cells of 14 individual healthy donors to lessen differences between donors (technique called MucilAir-Pool). The reconstituted airway epithelia was cultured at the air-liquid interface (ALI) in culture medium in 24-well plates with 6.5-mm Transwell inserts (cat #3470, Corning Incorporated, Tweksbury, USA). The cell cultures were stored 34 days at the air-liquid interface at start of experiments and were fully differentiated at the start of the experiment. A total number of 21 inserts was used in the experiment. Three days before the start of the experiment, the integrity and quality of all used cell culture inserts used was ensured by a three-step quality assurance test. Firstly, visual inspection under a conventional inverted microscope was performed. The pass criteria were visible cilia beating and uniform and homogenous appearance. Secondly, it was ensured that all inserts showed physiological mucus production by measuring the refractive index of the apical surface of the inserts. Thirdly, trans epithelial electronic resistance (TEER) was measured as described later in the description. Pass criteria was a TEER value of more than 200 Ω.cm². All inserts complied with the quality assurance test. Each insert was then, still on the same day, washed apically with culture medium to remove accumulated mucus and cell debris to minimize the risk of interference with the tests.

### Compositions

The compositions used in the experiment are specified in Table 1:

**Table 1: Compositions used in the experiment**

| Composition | Main component | Concentration of main component | Details of main component | Dose and application |
|---|---|---|---|---|
| Test 12.5 | Fucoidan | 1.25 mg/ml in normal saline | Fucoidan powder from *Undaria pinnatifida,* product Vesta UP, from Vesta ingredients, Inc. Average molecular weight: 150 kDa, purity: 95,68% fucoidan content, sulfate content: 30,04% | 10 µl, apically |
| Test 50 | As Test 12.5 | 5 mg/ml in normal saline | As Test 12.5 | 10 µl, apically |
| Test 100 | As Test 12.5 | 10 mg/ml in normal saline | As Test 12.5 | 10 µl, apically |
| Positive control | GS-441524 | 25µM in DMSO (final conc. of DMSO 0.05%) | Molecular weight: 291.26 Da | 500 µl, added to basal culture medium, and 10 µl normal saline apically |
| Negative control | Normal saline | / | / | 10 µl, apically |
| Positive control | Triton X-100 | 10% (v/v) in | - | 50 µl, apically |
| for cytotoxicity: Triton X-100 | | normal saline | | |

The test compositions were produced by mixing the fucoidan powder with normal saline (0.9% w/v NaCl in purified water) in a concentration of 10 mg/ml and solubilising by vortexing. This solution was further diluted with normal saline (0.9% w/v NaCl in purified water) by volume to obtain the test compositions of the concentrations 5mg/ml and 1.25 mg/ml. For each dose, a volume of 10 µl of test composition was pipetted onto the apical side of the cell model resulting in a dose of 12.5 µg or 50 µg or 100 µg fucoidan.

Investigational drug GS-441524 was provided by Epithelix and used as a positive control for inhibition of virus replication. GS-441524 is the active metabolite of the prodrug remdesivir. The drug was dissolved in 100% DMSO to make a stock solution of 50mM. This was diluted in medium to a final concentration of 25µM (and 0.05% DMSO). 500 µl of GS-441524 solution were added to the basal well 1 hour before viral inoculation. In previous studies it had been confirmed that this amount of DMSO has no impact on the parameters measured in the experiment. The basal medium was changed every day and the same dose of GS-441524 solution was subsequently added to the fresh basal culture medium. As a negative control, 10 µl normal saline were applied to the apical side of the cell cultures. Positive control for cytotoxicity was Triton X-100 (Polyethylene glycol tert-octylphenyl ether), a non-ionic surfactant often used in biochemical applications to permeabilise or lyse cell membranes. 50 µl at a concentration of 10% (v/v) in normal saline were pipetted onto the apical side of the inserts. Triton X-100 causes a massive LDH release in reconstituted human airway epithelia and was used as 100% cytotoxicity landmark.

### Virus inoculation

Coronavirus OC43 was isolated from clinical specimen in 2014 as described in Essaidi-Laziosi et al., 2017. Viral stocks for the experiments were produced in the MucilAir cell model by collecting apical washes with culture medium. The production of several days was pooled and quantified by qPCR, aliquoted and stored at -80°C. Inoculation of the cell cultures was performed on day 1 of the experiment. Prior to infection, the apical side of the inserts used for the experiment was washed once for 10 minutes with culture medium. Inoculations were performed with 100 µl of culture medium containing 10⁵ viral particles applied to the apical side of the cultures (the virus concentration was 10⁶/ml) and incubated for 3 hours at 34°C, 5 % CO₂. The viral solution used for the inoculation was re-quantified by qPCR and confirmed the viral genome copy number of the inoculum to be 1.1x10⁶/ml. Non-infected control inserts ("Mock") were exposed to 100 µl of culture medium without virus on the apical side for 3 hours at 34 °C, 5 % CO₂. Unbound viruses were washed away with culture medium after the 3 hours of incubation period by three rapid washing steps. Residual viruses after the 3 washes were collected by a 20 min apical wash and quantified by qPCR to establish a baseline for viral growth at later time points. Newly viral particles from replication in the infected cell cultures were collected by 20 min apical washes at 24, 48, 72 and 96 hours post-inoculation and quantified by qPCR.

### Test parameters

Viral genome copy numbers are a direct measure for the number of viral particles present in a sample. In the experiment, viral genome copy numbers of the cell cultures treated with the test compositions, positive control (GS-441524) and negative control (one not treated, one treated with culture medium) were compared to establish the effect of the test compositions on OC43 virus. To obtain the viral genome copy numbers, 20 µl of the 200 µl of apical washing liquid were used for viral RNA extraction with the QIAamp Viral RNA kit (Qiagen), obtaining 60 µl of eluted RNA. Viral RNA was then quantified by quantitative RT-PCR (QuantiTect Probe RT-PCR, Qiagen) using 5 µl of viral RNA with Mastermix and two OC43-specific primers and probe with FAM-TAMRA reporter-quencher dyes. Four dilutions of known concentration of OC43 RNA as well as control for RT-PCR were included and the plates were run on a Chromo4 PCR Detection System from Bio-Rad. Cycle threshold (Ct) data were reported to the standard curve, corrected with the dilution factor and presented as genome copy number per ml on the graphs.

Trans-epithelial electronic resistance (TEER) is a dynamic parameter that reflects the state of epithelia and is typically between 200 to 600 Ω.cm². An increase of the TEER value reflects a blockage of the ion channel activities of the MucilAir cell culture. A notable decrease of the TEER values, but with the value still above 100 Ω.cm² can be observed in certain cases and indicates an activation of the ion channels. Disruption of cellular junction or holes in the epithelia result in TEER values below 100 Ω.cm². When an epithelium is damaged, a decrease of TEER would be associated with an increase of LDH release or a decrease of the cell viability. In this example, TEER was measured after addition of 200 µl of culture medium to the apical compartment of the inserts (i.e. during the washing step). TEER was measured with an EVOMX volt-ohm-meter (World Precision Instruments UK, Stevenage) for each condition. Resistance values (Ω) were converted to TEER (Ω.cm²) using the following formula: TEER (Ω.cm²) = (resistance value (Ω) - 100 (Ω)) x 0.33 (cm²), where 100 Ω is the resistance of the membrane and 0.33 cm² is the total surface of the epithelium in one insert. The TEER measurement was conducted as follows: 200µl of culture medium was added on apical surface of each insert. The EVOMX was turned on. The electrode was washed with ethanol 70%, and the EVOMX screen shows the value -1. Then, the electrode was washed with culture medium, and the EVOMX screen shows 0.00. Then, the resistance (Ω) was measured with the EVOMX in the Ohms measurement function.

Lactate dehydrogenase (LDH) is a stable cytoplasmic enzyme that is rapidly released into the basal culture medium upon rupture of the plasma membrane. In the experiment, it served as a parameter indicating cytotoxic effect of the applied compositions. It was measured using the Cytotoxicity LDH Assay Kit-WST (Dojindo, CK12-20) which was read out using a plate reader to measure the absorbance of the samples at 490 nm. Samples were the basal media collected from all inserts at T48h and T96h. To determine the percentage of cytotoxicity, the following equation was used (A = absorbance values): Cytotoxicity (%) = (Aₓ (determined absorbance of the sample)-A_{L} (absorbance of the low control)/A_{H} (absorbance of the high control)-A_{L} (low control))^{∗}100. The high control was the positive control for cytotoxicity (10 % Triton X-100 apical treatment). Triton X-100 causes a massive LDH release and corresponds to 100 % cytotoxicity. The low control was basal culture medium of fresh MucilAir cell inserts. The negative controls (non-treated and vehicle) show a low daily basal LDH release, <5 %, which is due to a physiological cell turnover in MucilAir.

Cilia beating frequency (CBF) is a parameter that indicates if the nasal epithelia is healthy and carrying out its physiologic function of mucus transport. CBF was measured by a dedicated setup for this purpose. The system consists of three parts: a Sony XCD V60 camera connected to an Olympus BX51 microscope, a PCI card and a specific package of software. The cilia beating frequency is expressed in Hz. For measurement, a cell culture insert was placed under the microscope and 256 images were captured at high frequency rate (125 frames per second) at 34°C. CBF was then calculated using relevant software. It should be pointed out that CBF values may be subject to fluctuations due to parameters such as temperature, mucus viscosity or liquid applied on the apical surface of the cell model and observed values should be compared to a control condition in each experiment.

Mucociliary clearance (MCC) results from synchronised cilia-beating and also is a parameter that indicates if the nasal epithelia is healthy and carries out its physiologic function of mucus transport. MCC was monitored using a Sony XCD-U100CR camera connected to an Olympus BX51 microscope with a 5x objective. Polystyrene microbeads of 30 µm diameter (Sigma, 84135) were added on the apical surface of the cell cultures. Microbeads movements were video tracked at 2 frames per second for 30 images at 34°C. Three movies were taken per insert. Average beads movement velocity (µm/sec) was calculated with the ImageProPlus 6.0 software.

### Experimental protocol

The experimental setup was as below in Table 2. T specifies the point in time with the number indicating the number of hours after the start of the experiment, e.g. T24h specifies the time point 24 hours after start of the experiment.

**Table 2: Experimental setup of the experiment**

| Name of the test series | Number of repeats | Viral infection | Treatment at | | | | | | | | |
|---|---|---|---|---|---|---|---|---|---|---|---|
| | | | T0h | T4h9 | T8h | T24h | T32h | T48h | T56h | T72h | T80h |
| Mock | 3 | no | / | / | / | / | / | / | / | / | / |
| Negative control | 3 | yes | 10 µl | 10 µl | 10 µl | 10 µl | 10 µl | 10 µl | 10 µl | 10 µl | 10 µl |
| Test 12.5 | 3 | yes | 12.5 µg fucoidan | 12.5 µg fucoidan | 12.5 µg fucoidan | 12.5 µg fucoidan | 12.5 µg fucoidan | 12.5 µg fucoidan | 12.5 µg fucoidan | 12.5 µg fucoidan | 12.5 µg fucoidan |
| Test 50 | 3 | yes | 50 µg fucoidan | 50 µg fucoidan | 50 µg fucoidan | 50 µg fucoidan | 50 µg fucoidan | 50 µg fucoidan | 50 µg fucoidan | 50 µg fucoidan | 50 µg fucoidan |
| Test 100 | 3 | yes | 100 µg fucoidan | 100 µg fucoidan | 100 µg fucoidan | 100 µg fucoidan | 100 µg fucoidan | 100 µg fucoidan | 100 µg fucoidan | 100 µg fucoidan | 100 µg fucoidan |
| Positive (antiviral) control | 3 | yes | X µg GS-441524 basal + 10 µl normal saline apically | / | / | X µg GS-441524 basal + 10 µl normal saline apically | / | X µg GS-441524 basal + 10 µl normal saline apically | / | X µg GS-441524 basal + 10 µl normal saline apically | / |
| Positive control cytotoxicity (Triton X-100) | 3 | yes | 50 µl apically | / | / | 50 µl apically | / | 50 µl apically | / | 50 µl apically | / |

At the start of the experiment, T0h, all inserts were washed with 200 µl culture medium for 10 minutes at 34°C and the inserts were transferred into a new culture plate containing 500 µl of culture medium per well. According to the experimental setup, inserts were either treated with 10 µl of test composition in one of the three concentrations apically, with 10 µl negative control apically, with positive control from the basal side or not treated at all. All inserts were incubated for 1 hour.

At T1h, all inserts except the mock inserts were inoculated as described above. All inserts were incubated for 3 hours.

At T4h, all inserts were washed with three quick washing steps with 200 µl of culture medium at 34°C to remove the viral inoculum and unbound viruses. Afterwards, 200 µl of culture medium was added apically to all inserts and inserts were let sit for 20 minutes at 34°C. This apical liquid was then removed and was stored at -80°C until the virus copy number was determined. According to the experimental setup, inserts were either treated with 10 µl of test composition in one of the three concentrations apically, treated with 10 µl negative control apically, or not treated. All inserts were incubated for 5 hours.

At T8h, according to the experimental setup, inserts were either treated with 10 µl of test composition in one of the three concentrations apically, treated with 10 µl negative control apically, or not treated. All inserts were incubated for 16 hours.

At T24h, 200 µl of culture medium was added apically to all inserts and inserts were let sit for 20 minutes at 34°C. During this time, TEER of all inserts was measured. This apical liquid was then removed and was stored at -80°C until the virus copy number was determined. According to the experimental setup, inserts were either treated with 10 µl of test composition in one of the three concentrations apically, treated with 10 µl negative control apically, or not treated. The basal culture medium was removed and stored. All inserts were transferred to new culture plates containing 500 µl culture medium per well. According to the experimental setup, positive control inserts were again treated with positive control from the basal side. All inserts were incubated for 8 hours.

At T32h according to the experimental setup, inserts were either treated with 10 µl of test composition in one of the three concentrations apically, treated with 10 µl negative control apically, or not treated. All inserts were incubated for 16 hours.

At T48h, 200 µl of culture medium was added apically to all inserts and inserts were let sit for 20 minutes at 34°C. During this time, TEER of all inserts was measured. This apical washing liquid was removed and was stored at -80°C until the virus copy number was determined. 50 µl of the basal culture medium of all inserts was used for the LDH assay. All inserts were transferred to new culture plates containing 500 µl culture media per well. According to the experimental setup, inserts were either treated with 10 µl of test composition in one of the three concentrations apically, treated with 10 µl negative control apically, or not treated or were again treated with positive control from the basal side. All inserts were incubated for 8 hours.

At T56h, according to the experimental setup, inserts were either treated with 10 µl of test composition in one of the three concentrations apically, treated with 10 µl negative control apically, or not treated. All inserts were incubated for 16 hours.

At T72h, 200 µl of culture medium was added apically to all inserts and inserts were let sit for 20 minutes at 34°C. During this time, TEER of all inserts was measured. This apical washing liquid. was then removed and was stored at -80°C until the virus copy number was determined. According to the experimental setup, inserts were either treated with 10 µl of test composition in one of the three concentrations apically, treated with 10 µl negative control apically, or not treated. The basal culture medium was removed and stored. All inserts were transferred to new culture plates containing 500 µl culture medium per well. According to the experimental setup, positive control inserts were again treated with positive control from the basal side. All inserts were incubated for 8 hours.

At T 80h, according to the experimental setup, inserts were either treated with 10 µl of test composition in one of the three concentrations apically, treated with 10 µl negative control apically, or not treated. All inserts were incubated for 16 hours.

At T96h, the ciliary beating frequency (CBF) and the mucociliary clearance (MCC) of all inserts were measured. 200 µl of culture medium was added apically to all inserts and inserts were let sit for 20 minutes at 34°C. During this time, TEER of all inserts was measured. This apical washing liquid was then removed and was stored at -80°C until the virus copy number was determined. The basal culture medium was removed and stored. 50 µl of the basal culture medium of all inserts was used for the LDH assay.

### Results

The OC43 viral genome copy number data were expressed as log 10 ((copies/ml) + 1). The number 1 was added to include values of 0 copies/ml. Based on the study design and data from hours 24 - 96, the analysis used a repeated measures analysis of variance (ANOVA) with composition and time as factors. Since the repeated measurements on the same inserts are correlated and exhibited varied variability, the analysis used Proc Mixed in SAS^{®} for Windows, Version 9.4 (Cary, NC; USA) with an unstructured covariance matrix. The assumption of normality was checked via examination of summaries of residuals. The analysis set statistical significance at p-value ≤ 0.05. Due to the statistically significant composition x hour interaction and to control for the effect of multiple comparisons at the 0.05 significance level, each composition was compared to the negative control using Dunnett's Test at each day slice. To assess whether compositions achieved a 3 log 10 reduction from negative control, Dunnett-adjusted one-sided 95% lower confidence limits on the difference from negative control (i.e., negative control - composition) were formed. JMP^{®} Statistical Discovery^{™}, Version 14.0.0 (Cary, NC; USA) was used to summarize the results via tables and graphs.

Reduction of viral genome copy number:
The OC43 replication curves of the negative control, Test 12.5, Test 50, Test 100 and positive control inserts are given in Figure 1 and Table 3.

**Table 3: Results of the measurement of the viral genome copy number in example 1**

| | Mean | | | | | Standard Deviation | | | | |
|---|---|---|---|---|---|---|---|---|---|---|
| | Log 10 ((Copies/ml) + 1) | | | | | Loq 10 ((Copies/ml) + 1) | | | | |
| | Timepoint of measurement | | | | | Timepoint of measurement | | | | |
| Composition | T4h | T24h | T48h | T72h | T96h | T4h | T24h | T48h | T72h | T96h |
| Negative control | 0.00 | 5.39 | 7.77 | 8.29 | 7.99 | 0.00 | 0.47 | 0.48 | 0.36 | 0.10 |
| Positive antiviral control | 0.00 | 0.00 | 0.00 | 5.21 | 4.69 | 0.00 | 0.00 | 0.00 | 0.37 | 0.13 |
| Test 12.5 | 0.00 | 0.00 | 1.96 | 3.69 | 4.01 | 0.00 | 0.00 | 1.70 | 0.26 | 0.19 |
| Test 50 | 0.00 | 0.00 | 0.00 | 2.38 | 3.25 | 0.00 | 0.00 | 0.00 | 2.07 | 0.15 |
| Test 100 | 0.00 | 0.00 | 0.00 | 3.15 | 3.36 | 0.00 | 0.00 | 0.00 | 0.18 | 0.01 |

When the viral genome copy number was below the detection limit of the method, it was noted as 0.00 in the table and expressed as log 10 ((copies/ml) + 1) in the graphs. The number 1 was added to include values of 0.00 copies/ml in the graphs. As can be seen from these results and Fig.1, OC43 showed good viral replication in the negative control indicating that the cell model is suitable to test efficacy of the test compositions against OC43. In comparison to negative control, treatment with the positive antiviral control and all three concentrations of the fucoidan test composition statistically significantly reduced the apical genome copy number in the cell model at each timepoint (T24h - T96h). These reductions from negative control by the test compositions were statistically significantly greater than three log scales on days 1 - 4, with the exception of Test 12.5 at T72h. Additionally, on days 1-4, the apical genome copy numbers for all three concentrations of test composition were numerically less than or equal to the corresponding number for positive antiviral control except for Test 12.5 at T48h.

Coronaviruses utilise the membrane bound spike protein to bind to a host cell surface receptor to gain cellular entry. For example, the receptor binding domain (RBD) of the S1 domain of the spike protein (S protein) of SARS-CoV-2 binds to the cellular ACE2 receptor, while the RBD of the S protein of OC-43 binds to 9-O-acetlyated sialic acid. Without being bound to a specific theory, the present inventors believe that fucoidan, comprising sulfate groups negatively charged in solution and the physiological environment, interacts with positively charged surface proteins such as the receptor binding domain (RBD) of the S1 domain of the spike protein (S protein) of SARS-CoV-2 and OC-43 and thereby hinders the virus from entering the human epithelial cell. Whilst the tests described in the examples have been carried out with the coronavirus species OC43, the results can be extrapolated to other coronavirus strains having a similar positive net charge in RBD of the spike protein. Particularily, similar results are expected in testing against SARS-CoV-2 and Sars-Cov1. The RBD of OC43 comprises amino acids 327 to 541, comprising 21 basic amino acids and 15 acidic amino acids. The RBD of SARS-CoV-2 comprises amino acids 318 to 541, comprising 22 basic amino acids and 16 acidic amino acids. The net charge of both RBDs is +6. The negatively charged sulfate groups of the fucoidan polymer can form salt bridges with these positively charged amino acid residues of the RBD, and the polymeric structure can embrace the surface of the virus and interact with a plurality of S proteins on the viral surface and therefore block it from interacting with the epithelial cell glucosaminoglycans or receptors. The RBD may even undergo conformational change upon fucoidan binding, resulting in an inactivation of the S protein for receptor binding. Inactivated viral particles cannot infect the epithelial cells and cannot replicate.

### TEER:

All cell culture inserts at all measured timepoints showed TEER values from 800 to 300 Ω.cm², which is in the normal range for the cell model, except for the positive controls for cytotoxicity, which showed TEER values below 100 Ω.cm². The results show that the test compositions do not influence tissue integrity of human nasal airway epithelia. This indicates that the pharmaceutical compositions may be used *in vivo* as nasal spray compositions for humans without negative effects on the nasal epithelia.

### Cytotoxicity

As per the measurement scheme used for cytotoxicity explained above, LDH levels of Triton X-100 treated inserts was set to be 100% cytotoxicity, and the LDH levels measured in all other inserts were put in relation to this value. All cell culture inserts treated (except for the positive control) at all measured timepoints showed less than 5% cytotoxicity. This result shows that the compositions do not have cytotoxic effects and may be used *in vivo* as nasal spray compositions for humans without negative effects on the nasal epithelia.

### Cilia beating frequency (CBF)

CBF of the Mock (not infected) inserts was 8.5 Hz which is in the normal range for the cell model. Negative control, positive control, Test 12.5, Test 50 and Test 100, all showed significant increase of CBF to about 15 Hz. This may be the response of the cell model to the viral infection or may be related to the repeated apical liquid addition in these inserts.

### Mucociliary clearance (MCC)

Negative, uninfected control (mock) showed beads' velocity of 4 µm/s at 34°C, which is unexpected and out of the normal range of the cell model (normal range: 40-50 µm/s). Repeated exposure to apical liquid, or OC43 infection increased the mucociliary clearance toward normal values, where OC43 infection treated with apical negative control was significantly different from Mock. Test compositions did not change significantly mucociliary clearance compared to negative control.

## Claims

1. Pharmaceutical composition comprising fucoidan for use in a method for the treatment or the prevention, by intranasal administration, of coronavirus infection in a human.

2. Pharmaceutical composition for use according to claim 1 wherein the coronavirus infection is an infection with OC43 or SARS-CoV-2 or SARS-CoV-1.

3. Pharmaceutical composition for use according to claim 2 wherein the coronavirus infection is an infection with SARS-CoV-2.

4. Pharmaceutical composition for use according to any of the preceding claims wherein the coronavirus infection is an infection with a coronavirus species which binds electrostatically to fucoidan.

5. Pharmaceutical composition for use according to any of the preceding claims wherein the coronavirus infection is an infection with a coronavirus species which has a positive net charge in a receptor binding region.

6. Pharmaceutical composition for use according to claim 5 wherein the coronavirus infection is an infection with a coronavirus species that has a positive net charge of at least 5 in a in a receptor binding region of the spike protein.

7. Pharmaceutical composition for use according to any of the preceding claims wherein the composition is a nasal spray.

8. Pharmaceutical composition for use according to any of the preceding claims wherein the pharmaceutical composition is an aqueous solution.

9. Pharmaceutical composition for use according to any of the preceding claims wherein the composition comprises 0.1% to 2.7% (w/w), preferably 0.25% to 1.8%, most preferably 0.5 to 1% (w/w) fucoidan.

10. Pharmaceutical composition according to claim 9 wherein the pharmaceutical composition comprises a sodium chloride solution.

11. Pharmaceutical composition for use according to any of the preceding claims wherein the aqueous solution is isotonic.

12. Pharmaceutical composition for use according to any of the preceding claims wherein the pharmaceutical composition is administered intranasally two or three times daily into each nostril of a subject.

13. Pharmaceutical composition for use according to any of the preceding claims wherein the pharmaceutical composition is administered in a dose of 3.75 mg to 10.15 mg fucoidan into each nostril of a subject per administration.

14. Pharmaceutical composition for use according to any of the preceding claims, wherein the fucoidan is a fucoidan extracted from Undaria pinnatifida.

15. Pharmaceutical composition for use according to any of the preceding claims, wherein the fucoidan has an average molecular weight of 150 kDa and a lower molecular weight cut-off of 10 kDa.

16. Pharmaceutical composition for use according to any of the preceding claims, wherein the fucoidan has a sulfate content of 20% to 40%.

17. Pharmaceutical composition according to any of the preceding claims for use in a method for prevention of coronavirus spread in a human population, wherein subjects of the population are tested for infection with OC43 or SARS-CoV-2 and infected subjects are treated with intranasal administration of the pharmaceutical composition.

18. Pharmaceutical composition according to any of the preceding claims for use in a method for prevention of coronavirus spread in a human population wherein subjects who have been identified as being at a high risk of infection are treated with intranasal administration of the pharmaceutical composition.
